# EUROPEAN PATENT APPLICATION

(11) **EP 1 238 663 A2**
(43) Date of publication of application: **11.09.2002**
(21) Application number: 02380049.3
(22) Date of filing: 28.02.2002
(51) Int. Cl.: A61K 9/19, A61K 47/36, A61K 47/02

(54) **Mixed hydrophilic gels as controlled release vehicles or floating dispersants**

(30) Priority: 01.03.2001 ES 200100492
(71) Applicant: Industrial Farmaceutica Cantabria, S.A., 39011 Santander (ES)
(72) Inventor: Matji Tuduri, Jose Antonio, 28043 Madrid (ES); Pivel Ranieri, Juan Pablo, 28009 Madrid (ES); Gerrero Gomez-Pamo, Antonio, 28007 Madrid (ES); Escribano Arias, Marta, 28014 Madrid (ES); Dominguez Valdes-Hevia, Marta, 28043 Madrid (ES); Garcia Martinez, Fernando, 28250 Torrelodones (Madrid) (ES); De La Mata Maltzeff, Belen, 28660 Boadilla del Monte (Madrid) (ES); Gallo Molina, Ramon, 28020 Madrid (ES)
(74) Representative: Del Santo Abril, Natividad

(57) **Abstract**

It consists of two hydrophilic polymers with gelling properties, giving rise to two gels, in which one of them is capable of interacting with the active ingredient (catching gel or gel A), at least modulating its release, and the other has the function of homogeneously dispersing the first gel, and ensuring the floatability and adhesion of the complex system in the gastric cavity, and may also intervene in the modulation of the active ingredient release (dilution gel or gel B). The product that is the object of the invention consists of the intimate mixture of the two hydrophilic gels that in turn consists of polysaccharides. One of the hydrophilic polysaccharides is agar, whereas the second can be selected from either alginate or chitosan.

## Description

### BACKGROUND TO THE INVENTION

Orally administered controlled release systems are pharmaceutical formulations designed to increase the bioavailability of the active ingredients in systemic products and to eliminate the peaks and fluctuations in their plasmatic levels. In general, it can be said that the idea is to increase the regularity and predictability of the active ingredients' performance. Moreover, oral administration is evidently ideal for products targeted at the organs in the gastrointestinal tract (hereinafter, GIT). If, for example, the target organ is the stomach, the aim is a longer retention time and a homogeneous distribution of the active ingredient.

On the other hand, oral administration is extremely difficult. Although it is the simplest from a practical viewpoint, and therefore the most common, it is possibly the most complex, since it implies the interaction of the drug in the pharmaceutical formula with different mucosal or immunological systems, different pHs, the presence or absence of bacterial flora, etc. All this means that a specific design is required, depending on the point where the action or absorption is desired. The length of the system itself, which in humans is around 10 m, has also to be considered.

Moreover, the normal physiology of the GIT can often be, and in fact is, altered by the active ingredient vehicle (modified transit, for example).

One of the delayed action mechanisms that have been described is the floating system, as described, for example, for levodopa, using HBS® capsules.

In general, the idea is for the vehicle to be capable of floating in the gastric cavity, gradually releasing the active ingredient for systemic formulas, or facilitating its retention and homogeneous distribution if the stomach is the target organ. Therefore, the floatability of the different excipients can be initially related to an absorption modulation. The subject of the floatability of different excipients has been the subject of different bibliographical reviews, from which the following references can be selected:
(1) BN Singh, KH Kim "Floating drug delivery systems: an approach to oral controlled drug delivery via gastric retention." J Controlled Release 63, 235 - 259 (2000).
(2) HM Ingani, J Timmermans, AJ Moës "Conception and in vivo investigation of peroral sustained release floating dosage forms with enhanced gastrointestinal transit." Int J Pharmaceutics 35, 157 - 164 (1987).
(3) J Timmermans, AJ Moës "How well do floating dosage forms float?" Int J Pharmaceutics 62, 207 - 216 (1990).
(4) VS Gerogiannis, DM Rekkas, PP Dalas, NH Choulis "Floating and swelling characteristics of various excipients used in controlled release technology".
(5) O Smidsrod, KI Draguet "Chemistry and physical properties of alginates". Carbohyd in Europe Vol. 14, May 1996, pages 6 - 13.
(6) J Timmermans, AJ Moës "Factors controlling the buoyancy and gastric retention capabilities of floating matrix capsules: New data for reconsidering the controversy." J Pharmac Sci 83, 18 - 24 (1994).
(7) GA Digenis, EP Sandefer, RC Page, WJ Doll "Gamma scintigraphy: an evolving technology in pharmaceutical formulation development - Part 1". Pharm Sci Tech Today 1, 100 - 165 (1998).

Different polymers have been used in different floating formulations, including chitosans, alginates, agar, polylactic, polyacrylates, etc., all of which are polymers with different hydrophilicity, pH sensitivity, swelling speed, etc.

"Floating" vehicles are also especially suitable for aiming the active ingredient at the gastric cavity. When the target is another GIT organ, the systems have to be more complex regarding their specific organ recognition capacity.

### OBJECT OF THIS INVENTION

The object of this invention is the design of different orally administered pharmaceutical systems that simultaneously combine the properties of gel convertibility, floatability, controlled release, dispersability, adhesion, non-modification of the gastrointestinal transit, hydrophilicity, administrable free or in capsule form, and stability, and finally are capable of interacting well, retaining different types of drugs (anionic, cationic, neutral, etc.), to modulate the release of active ingredients after their systemic absorption or homogeneously disperse substances targeted at the gastric cavity itself. In this case, the idea is not to retain the active ingredient by interaction with a hydrophilic matrix floating in the gastric cavity, but a greater retention and homogeneous distribution of the active ingredient in the cavity itself.

### DESCRIPTION OF THIS INVENTION

To simultaneously comply with all the above requirements, binary systems have been designed, consisting of two hydrophilic polymers with gelling properties, giving rise to two gels, in which one of them is capable of interacting with the active ingredient (catcher gel or gel A), modulating at least partially its release, and the other has the function of dispersing the first gel homogeneously, and the floatibility and adhesion of the complex system in the gastric cavity, and it may also intervene in the modulation of the active ingredient release (dilution gel or gel B). These systems can be prepared so that their final form is humid, with the gels swollen, or dry. The latter is one of the ways of regulating the active ingredient's release, in so much as the dry gels absorb water and swell and the distribution is established between the active ingredient retained in the gel and the external aqueous medium.

The product or composition that is the object of this invention consists of the intimate mixture of two hydrophilic gels that in turn consist of polysaccharides. The mixture may or may not have been dried, and it has the following properties:
- One of the hydrophilic polysaccharides is agar (complex polysaccharide extracted from rhodophytan algae and formed by linked α (1-3) and β (1-4) galactose polysaccharide chains, which can be sulphated, acetylated, etc., as described, for example, in the Merck Index, 12^{th} ed., page 34).
- The other hydrophilic polysaccharide can be either of the following two:

- Alginate (polysaccharide consisting of residues of β-D-manosiluronate and α-L-gulusiluronate linked in 1-4, as described, for example, in the Merck Index, 12^{th} ed., page 45).
- Chitosan (polysaccharide derived from chitin and consisting of residues of β-D-glucosamine linked 1-4, as described, for example, in the Merck Index, 12^{th} ed., page 342).
- In its dry form it can be suspended in water or in aqueous solutions and it is capable of slowly absorbing water, giving rise to a mixture than can be dispersed simply by stirring, which has the characteristics described for the liquid suspension form.
- In its liquid suspension form it is capable of floating on liquids such as incomplete gastric juice (consisting of NaCl 2 g/L and concentrated HCl (density 1.19) 7 mL/L in de-ionised water), adhering to surfaces such as glass, catching substances (active ingredients) and release them progressively, or act as a dispersing agent for substances (active ingredients).
- The active ingredients concerned, which are incorporated into the process of obtaining the product or composition that is the object of this invention, are substances that are soluble or can be suspended in water or aqueous solutions, such as riboflavin 5 phosphate, paracetamol, dimemorfan, ibuprofen, gentamycin, cimetidine, etc.
- The dispersible active ingredients, which are also incorporated into the process of obtaining the product or composition that is the object of this invention, are substances that are insoluble or difficult to suspend in water, such as magaldrate and other neutralising anti-acids such as complex aluminium and magnesium salts, etc.
   This product or composition is obtained in accordance with the general procedure described below:
   The procedure consists of preparing two gels separately, which are intimately mixed by trituration when prepared.
      - The first gel, gel A or retention gel, has one hydrophilic polymer, agar, at a high concentration (between 0.4 and 1.8% w/w in the mixture forming gel A) and a second polymer (alginate or chitosan) (at a concentration of 0.02 and 1.0% w/w in the mixture forming gel A), and an important feature of the system is that although the second polymer is capable of gelling, the relevant gelling agent is found in the second gel, gel B or dilution gel. This gel A has a greater retention capacity, due to the sieve effect and ionic interaction.
   - The second gel, gel B or dilution gel, consists of agar at a low concentration (between 0.04 and 0.1% w/w in the mixture forming gel B) and the gelling reagent or reagents of the second polymer of gel A, which can be, for example, calcium chloride for alginate (at a concentration of ½ and 1/8 in relation to the alginate) and sodium tripolyphosphate for chitosan (at a concentration of between ¼ and 1/8 of the chitosan), with the properties of dispersing the first gel and gelling its second component when both gels are mixed.

Both gels are formed separately, in both cases first dissolving the agar in a very hot aqueous solution (at a temperature of over 80°C) and then lowering the temperature (not below 40 - 45°C), and adding the rest of the components; finally, they are left to gel by incubation at a low temperature (below 30°C). Once both gels have been formed separately, they are roughly triturated, mixed, and then exhaustively triturated, facilitating the interaction between the gelling ingredient in gel B and the second polymer in gel A. Once this internal mixture of the two gels is complete, it is left to rest for a time at a low temperature for the mixed gel (A + B) to form. After this time, which will depend on the polymer concentrations and the possible effects of the molecules on the gelling, it is exhaustively triturated and, optionally, depending on the final pharmaceutical presentation, it is dried by freeze-drying or another suitable method.

The active ingredient can be included in gel A or B, depending on factors such as the ion load, solubility, interference with the gelling process, etc., and objective (catching or dispersion). The concentration will also depend on these factors, and can be between 0 and 20% w/w in the mixture forming the respective gel.

### DETAILED DESCRIPTION OF THE INVENTION: EXAMPLES

### EXAMPLE 1

### PREPARATION OF THE SYSTEM THAT IS THE OBJECT OF THIS INVENTION, EMPTY (WITH NO MOLECULES)

Following is a description of how to prepare the controlled release system without vehicle molecules. As described, it is manufactured in two phases, with a different polymer concentration in each phase, fraction A and fraction B. Once both bases have been prepared, they are triturated and mixed in equal quantities.
Manufacture of fraction A (formula for 150 g w/w):
1^{st}.- Each of the products is weighed: Agar-agar 1.4 g, sodium alginate 0.6 g, purified water 148 g.
2^{nd}.- 140 g of purified water is heated to boiling point in a 300 mL glass beaker, in heated magnetic stirring equipment.
3^{rd}.- When it reaches boiling point, the Agar-agar is added gradually.
4^{th}.- When dissolved, stirring and heating continues for five minutes.
5^{th}.- The heating is switched off and stirring continues until the temperature falls to 70°C.
6^{th}.- When the temperature reaches 701C, the sodium alginate is added gradually, continuing to stir until it is completely dissolved.
   (When an active ingredient is to be added to this fraction (see following examples), it is added when, after the alginate is completely dissolved, the temperature has fallen at least to 60°C (or the highest temperature that the active ingredient can support, providing it is no lower than 40 - 45°C).
7^{th}.- When the components are dissolved, it is completed to 150 g with purified water.
8^{th}.- It is left to gel in a refrigerator (at approximately 4 - 10°C).
   Manufacture of fraction B (formula for 150 g w/w):
1^{st}.- Each of the products is weighed: agar-agar 0.15 g, calcium chloride 0.1 g, purified water 149.75 g.
2^{nd}.- The purified water is heated to boiling point in a 300 mL glass beaker, in heated magnetic stirring equipment.
3^{rd}.- When it reaches boiling point, the Agar-agar is added gradually.
4^{th}.- When dissolved, the stirring and heating continues for five minutes.
5^{th}.- The heating is switched off and stirring continues until the temperature falls to 50°C.
6^{th}.- When the temperature has reached 50°C, the calcium chloride that was previously dissolved in water is added (adding 3 mL of a 3% solution in purified water), continuing to stir.
   (When the active ingredient is to be added to this fraction (see following examples), it is added when, after adding the calcium chloride, the temperature has fallen to at least 50°C (or the maximum temperature supported by the active ingredient, providing it is no lower than 40 - 45°C).
7^{th}.- It is completed to 150 g with purified water.
8^{th}.- It is left to gel in a refrigerator (at approximately 4 - 10°C).
   Manufacture of the mixture of fractions A and B.
1^{st}.- Once both fractions have gelled, fraction A, previously roughly broken up with a spatula, and fraction B are put into a 500 ml glass beaker.
2^{nd}.- On scales, the weight is adjusted to 300 g with purified water.
3^{rd}.- The gels are triturated with a Silverson mixer. The mixer head is inserted approximately 1 cm above the liquid. It is then mixed at a medium speed until the pieces disappear. The product is left to rest for around 10 minutes, and then it is mixed again for 10 minutes with the mixer head on the bottom of the beaker at the same speed.
4^{th}.- It is left to rest in the covered beaker, in a refrigerator (at approximately 4 - 10°C) for 12 - 24 hours.
5^{th}.- It is triturated at ambient temperature in a Silverson mixer for 10 minutes, with the mixer head on the bottom of the beaker, and at low speed.
   Preparation of the freeze-dried product:
   This process takes place 48 hours after completing the manufacture of the product in the refrigerator.
1^{st}.- The product is introduced into freeze-drying chambers, so that it occupies no more than 1/5 of the volume of the chamber.
2^{nd}.- The chambers are frozen to -80°C, and the content is left to freeze (approximately 20 - 30 minutes).
3^{rd}. - It is freeze-dried in a Teistar Cryodos or similar freeze-dryer (condenser temperature -35°C to -40°C, approx. vacuum 1 µ).
4^{th}.- When freeze-dried, it is removed from the freeze-dryer and stored at a low temperature (4°C).

The freeze-drying process can be replaced by another drying process, depending on the stability of the substance concerned.

### "IN VIVO" BEHAVIOUR OF THE SYSTEM

Following is a description of the floating behaviour of the system that is the object of this invention (FDS system) in pigs, with gammagraphy techniques based on the product marked as ^{99m}Tc (according to procedures described, for example, in SS Davis, JG hardy, SP Newman, IR Wilding "Gamma scintigraphy in the evaluation of pharmaceutical dosage forms". Eur J Nuc Med 19, 971 - 986 (1992).

FDS System marking
1.- FDS System marking with the radionuclide ^{99m}Tc (according to the technique described in J. Steigman, G Meinken, P Richards "The reduction of pertechnetate-99 by stannous chloride. The stoichiometry of the reaction in HCl, in citrate buffer and Ind DTPA buffer". Int J App Radiat Isotopes 26, 601 - 609 (1975)):
   The FDS System was marked directly through ionic bonds based on a master solution containing the component to be marked (8 mg/mL in water) and an acid tin solution as a technetium-reducing agent. The proportions of the components of the tin solution, and the incubation times, were modified until a good marking efficiency (78 - 86%) was obtained with the best possible stability conditions.
2.- Determination of the radiochemical purity and stability of the FDS System by ascending chromatography and centrifugation, measuring the radioactivity in the supernatant (^{99m}Tc) and in the precipitate (FDS System - ^{99m}Tc). It was then dialysed for 6 hours in order to remove the radiochemical impurities (free technetium) prior to the "in vivo" tests.

### "In vivo" tests

The objective was to evaluate the FDS System marked with ^{99m}Tc by means of isotopic studies of gastric emptying in an experimental animal model using pigs of the Landrace Large white species suitable for extrapolating the experimental results to humans. Three experiments (2 isotopic dynamic gastric emptying studies) were performed with each animal:
1.- Negative Control: To determine the gastric emptying standard and the normal reference values for each of the experimental animals.
2.- Marked FDS System in a total of 8 animals.
3.- Negative control with non-marked FDS System in order to verify that the FDS System does not alter gastric emptying "per se".

To evaluate gastric emptying, the animals were positioned on their backs in a dual-head gamma chamber, acquiring dynamic dorsal and ventral images throughout the study (7 hours).

Upon completion of the study, we obtained the gastric emptying curves (activity/time curves corrected by the radioactive decay of the radionuclide ^{99m}Tc) to determine:
A) Standard gastric emptying for liquids in the experimental animals.
B) Standard gastric emptying for the product, analysing: The product's gastric behaviour, if there are changes regarding gastric emptying compared with the control and the time that the product remains in the stomach.

To evaluate the results, we calculated the following variables from the disintegration per second corrected by the "radioactive decay" in pre-established times (every 15 minutes, from the start to the end of the study): The % of gastric retention activity throughout the study, Area: average activity per 15 minutes, Accumulated area:
accumulated average activity per 15 minutes, Possible area: the area of the curve (variable determined from the area and accumulated area variables).

The results obtained were as follows.
1. The half time (time in which half of the activity was eliminated) was 18 minutes for the control group, whereas in the marked FDS System group, half of the activity had still not been eliminated after 4 hours.
2. The percentage of average activity that remains in the stomach was 24% and 30% for the control group and the FDS System-control group, respectively, after one hour, and 11 and 12% respectively after four hours, whereas in the studies conducted with the marked FDS System, it was 73% after one hour, 62% after four hours and 47% after 7 hours.
3. The total area under the curve expressed as a proportion of the possible area was 0.24 and 0.28 for the control group and the FDS System-control group, respectively, compared with 0.7 for the FDS System group.

From these results, we can conclude that:
1. There are statistically significant differences (p<0.001) between the control group and the FDS System group, both in the percentage of activity and in the possible area at all the time intervals.
2. The control group and the FDS System-control group have a similar gastric emptying pattern, and there are no statistically significant differences between them.
3. The marked FDS System remains in the stomach 7 hours after administration, unlike the control group.
4. The FDS System "per se" does not alter the gastric dynamics.

The FDS System described in the previous example can be used to contain or disperse different active ingredients, as shown in the following examples:

### EXAMPLE 2

### PREPARATION OF THE SYSTEM THAT IS THE OBJECT OF THIS INVENTION, TO CONTAIN PARACETAMOL (FDS System-Paracetamol).

The FDS System-paracetamol is prepared following the procedure described in example 1, adding the paracetamol:
a) in equal quantities to fractions A and B to give a final content of 1% w/w in the finished mixture.
b) Adding the paracetamol to fraction A to give a final content of 0.25% w/w in the finished mixture.
c) Adding the paracetamol to fraction A to give a final content of 0.50% w/w in the finished mixture.

### RELEASE STUDIES ON THE PARACETAMOL IN FDS SYSTEM-PARACETAMOL

The release studies were performed by dialysis at 37°C against artificial gastric juice without pepsin (7 ml HCl with d 1.19 and 2 g NaCl in 1 l of MilliQ water and with the System FDS-Paracetamol product previously reconstituted with MilliQ water at concentrations of between 5 and 20 mg/ml, using as a reference free paracetamol at a concentration of 10 mg/ml in MilliQ water.

The paracetamol evaluations were conducted with a standard curve for paracetamol at 100 mg/ml in MilliQ water determined by spectroscopic absorbency evaluation at 243 nm.

A delayed release effect is produced especially in the preparations in which the concentration of paracetamol in the mixture prepared was between 0.25 and 0.5% (w/w) and with a release speed of 75% compared with the control, so the total release of the dose occurred in 2.0 - 2.5 hours (less than the time that the vehicle remained in the stomach).

### EXAMPLE 3

### PREPARATION OF THE SYSTEM THAT IS THE OBJECT OF THIS INVENTION CONTAINING RIBOFLAVIN 5 PHOSPHATE (FDS System-Riboflavin).

The FDS System-riboflavin is prepared following the procedure described in example 1, adding the riboflavin 5 phosphate in equal quantities to fractions A and B up t a final content of 0.1% w/w in the mixture. Once the freeze-dried FDS System-riboflavin has been obtained, it is packed in hard gelatine capsules with a content of riboflavin 5 phosphate per capsule of 6 mg.

### RELEASE STUDIES ON THE RIBOFLAVIN 5 PHOSPHATE IN FDS SYSTEM-RIBOFLAVIN.

It is conducted using the FDS System-Riboflavin product in capsules, studying the release into artificial gastric juice (as in example 2) at 37°C and with free riboflavin 5 phosphate in gelatine capsules as a reference. The riboflavin 5 phosphate is quantified spectrofluorimetrically (exciting to 267 ± 5 nm and reading the emission at 520 ± 5 nm), interpolating to the relevant reference line.

In the described conditions, FDS releases 25% of the Riboflavin after 3 hours, whereas release control is total after 15 minutes.

### EXAMPLE 4

### PREPARATION OF THE SYSTEM THAT IS THE OBJECT OF THIS INVENTION CONTAINING IBUPROFEN (FDS System-Ibuprofen)

The FDS System-Ibuprofen is prepared following the procedure described in example 1, adding the Ibuprofen to fraction A up to a final content of 6.6% w/w in the mixture.

### RELEASE STUDIES ON THE IBUPROFEN IN FDS SYSTEM-IBUPROFEN.

They are conducted studying the release of the ibuprofen or in phosphate buffer 20 mm pH 7.4 at 37°C and with free ibuprofen as a reference. The ibuprofen is quantified spectrophotometrically by absorbency at 265 nm, interpolating to the relevant reference line.

In the test conditions, the ibuprofen is released gradually, releasing 75% of the content in 2 hours, whereas the free ibuprofen is dissolved in 5 minutes in the buffer.

### EXAMPLE 5

### PREPARATION OF THE SYSTEM THAT IS THE OBJECT OF THIS INVENTION CONTAINING GENTAMYCIN (FDS System-Gentamycin)

The FDS System-Gentamycin is prepared following the procedure described in example 1, adding the Gentamycin to fraction B up to a final content of 0.8% w/w in the mixture.

### STUDIES ON THE BINDING OF THE GENTAMYCIN TO THE FDS SYSTEM.

The release of the Gentamycin from FDS System-Gentamycin is studied by introducing the FDS System-Gentamycin into a glass column (like a chromatographic support) and eluting well with phosphate buffer 20 mm pH 7.4 or with artificial gastric juice (following example 2). The gentamycin is evaluated after reacting with ortho-phtaldehyde and HPLC separation with C18 column, detecting at 330 nm.
In the test conditions, the gentamycin is not released from the FDS System-Gentamycin with phosphate buffer and over 3 volumes of gastric juice are required to elute it, which proves its interaction with the system.

### EXAMPLE 6

### PREPARATION OF THE SYSTEM THAT IS THE OBJECT OF THIS INVENTION, DISPERSING MAGALDRATE (FDS System-Magaldrate).

As described in the General Description of the invention, the FDS System not only obtains a delayed effect from the preferred binding to a mixed hydrophilic gel with floating/retaining capacity but it also optimises pharmaceutical formulations in which the therapeutic target is a component of the GIT, concretely the stomach. For neutralising anti-acids, the fundamental difference with the other active ingredients tested is that the quantity of the active ingredient to be contained is much greater, and therefore the mission of the mixed FDS hydrophilic gel is, more than to retain, to disperse the neutralising agent to obtain its homogeneous distribution in the gastric cavity, thus optimising the use of the neutralisation equivalents of the neutralising agent.

The FDS System-Magaldrate is prepared as follows:
A) 355 g and 200 g are obtained of fractions A and B, respectively, as described in example 1, adding magaldrate to fraction A up to a final concentration of 10% (w/w of the mixture obtained) and to fraction B up to a final concentration of 18% (w/w of the mixture obtained), leaving them to gel as indicated in example 1.
B) 125 g of a fraction consisting of an aqueous magaldrate suspension at 19% w/w is prepared.
C) Fractions A, B and C are mixed and processes as indicated in example 1 for the mixture of fractions A and B. The mixture of the three is the final product, which is not freeze-dried (in the final preparation and before processing, a CO₂ generator such as Na₂CO₃ may or may not be added).

### EVALUATION OF THE NEUTRALISING SPEED OF FDS SYSTEM-MAGALDRATE COMPARED WITH THE NEUTRALISING SPEED OF MAGALDRATE

The FDS System-Magaldrate is capable of neutralising the same quantity of hydrochloric acid "in vitro", for equal quantities of active ingredient, at a speed between 10 and 20 times greater than Magaldrate, which shows the efficacy of the active ingredient dispersion.

### PHARMACOLOGICAL EVALUATION OF THE NEUTRALISING CAPACITY OF FDS SYSTEM-MAGALDRATE COMPARED WITH CONTROL MAGALDRATE

The neutralising capacity of the FDS System-Magaldrate formulation was evaluated "in vivo" in a model with pigs (Landrace large white) between 16 and 18 kilos in weight. Intragastric acidity was evaluated with a digital pH-meter connected to a calibrated pH catheter with two sensors 5 cm apart, which detects the pH in two different sectors of the stomach. For the evaluation of the pH, a fistula was performed between the stomach and the abdominal cavity. Baseline and further studies were conducted over three days on each animal and on the test date, the animals received the anti-acid product by probe. The products were: placebo, reference magaldrate and FDS System-magaldrate. The animals fasted from the day before the test. The intragastric pH was recorded continuously for 12 hours (1 reading every 4 seconds).

The results obtained are expressed as "neutralising time", which is defined as the time taken to return to the initial pH. The mean values were 47 minutes for the reference magaldrate and 110 minutes for the FDS System-magaldrate. This result confirms, in an animal model that can be extrapolated to humans, the pharmacological optimisation obtained by the use of the FDS System.

## Claims

1. A composition for pharmaceutical use for oral administration, consisting of two hydrophilic polymers of a saccharide nature capable of gelling, in which the two gels are formed independently and then roughly triturated and mixed and then exhaustively triturated, giving rise to a pharmaceutical vehicle that can be used humid or dry. The first gel (gel A or retention gel) consists of agar at a high concentration and a second polymer capable of gelling. The second gel (gel B or dilution gel) consists of agar at a low concentration and the gelling reagent of the second component of the first gel.

2. A composition according to claim 1, in which the second component of the first gel (gel A) can be a type of alginate or chitosan.

3. A composition according to claim 1, in which the component of gel B, the chemical gelling agent of the second component of the first gel, is either calcium chloride or sodium tripolyphosphate, respectively.

4. A composition according to claim 1 in which the active ingredient to be contained can be added prior to the gelling to the hard gel (gel A or retention gel), to the soft gel (gel B or dilution gel) or to both, depending on the particular interactions of the active ingredient concerned with the polymers to be gelled.

5. A composition according to claim 1 in which, when the active ingredient is an anti-acid, it can be incorporated to both gels, and free anti-acid may or may not be incorporated into the pharmaceutical formulation.

6. A composition according to claim 1 in which the active ingredient to be contained can be a non steroidal anti-inflammatory drug such as ibuprofen, an analgesic such as paracetamol, molecules containing amino groups such as aminoglycosides or central analgesics, cimetidine and similar H2 antagonists.

7. A composition according to claim 1 in which once the active ingredient has been added, the final product may be maintained humid or dry.

8. A procedure for manufacturing the composition according to claim 1 in which gel A and gel B are roughly triturated and then mixed, and then this normal mixture is intimately mixed by trituration with a Silverson type machine, and in which this intimate mixture is left to rest at a low temperature to facilitate the interaction between the second component of gel A and the gelling component in gel B.

9. A procedure according to claim 8 in which the resting temperature can be between 4 and 14°C.

10. A procedure according to claim 8 in which the resting time can be between 8 and 36 hours.

11. A procedure according to claim 8 in which the active ingredient to be contained can be incorporated into to the retention gel (or gel A) or the dilution gel (or gel B) or both.

12. A procedure according to claim 8 in which the final composition can be dehydrated by a procedure suitable for the active ingredient contained, such as freeze-drying or dissecation, atomisation, etc.

13. A pharmaceutical product according to composition 1 and according to claim 5 with an active ingredient (neutraliser) in a therapeutically effective quantity.

14. A pharmaceutical product according to composition 1 and according to claim 6 with the active ingredient in a therapeutically effective quantity.

15. A pharmaceutical product according to claim 1 in which the final pharmaceutical presentation can be in gel form, or when the product is dissecated, in capsule, tablet or sachet form.
